# EUROPEAN PATENT APPLICATION

(11) **EP 1 886 664 A1**
(43) Date of publication of application: **13.02.2008**
(21) Application number: 06016517.2
(22) Date of filing: 08.08.2006
(51) Int. Cl.: A61K 8/46, A61Q 11/00, A61Q 11/02, C11D 3/00

(54) **N-chloroamines containing compositions**

(71) Applicant: Pereira, José Sidnei, Cornélio Procópio, PR (BR); Landi, Flávio, Cornélio Procópio, PR (BR)
(72) Inventor: Pereira, José Sidnei, Cornélio Procópio, PR (BR); Landi, Flávio, Cornélio Procópio, PR (BR)
(74) Representative: Jennings, Tara Romaine

(57) **Abstract**

A composition that is formulated as a dental paste, a mouthrinse, a therapeutic gel, a preserving solution or a cleansing cream comprising an antiseptic agent in an aqueous solution selected from the N-chloramines and an anionic surfactant agent.

## Description

The present invention discloses organic systems that are formulated as dentifrices in the form of a paste or gel, a mouthrinse, a therapeutic gel, a preserving and cleansing solution, a cleansing cream for removable dentures with an active principle of antiseptic and biocide properties selected from the N-chloramines, like T-chloramine, or any other compound derived from N-chloramines that associated with other compounds impart to the systems certain properties as biocide, anticaries, anti-inflammatory, preserving, curing, teeth plaques antibacterial, anti-tartar, halitosis deodorizing, dental desensitizing and cleansing, providing to the organic systems unmatched conditions in the preservation and therapy of several oro-buccal diseases and as well providing the same unmatched conditions to the individual's mouth cleansing care. As mentioned before, formulations basically comprise an antiseptic agent and biocide in an aqueous solution selected from the N-chloramines preferably the T-chloramines, such agent added to the mass of one of systems (I) with a concentration (CII); expressed in a percentage (%) of weight in relation to the total mass (I+II+III), CII in a maximum of 4%, preferably CII 2% and, more preferably CII in a minimum of 0.01% up to or equal to 1%; III an anionic surfactante agent selected preferably in a group of tensoactive formulations as lauryl diethyleneglycol ether sodium sulfate, more preferably lauryl sodium sulfate added to the mass of one of the systems I with a concentration CII expressed in a percentage (%) of weight in relation to the total mass (I + II + III). CIII in a maximum of percentage (%) preferably CIII 2% and, more preferably a minimum of 0.01% up to or equal to 3%.

### FUNDAMENTALS OF THE TECHNIQUE

Since the very beginning the human being suffer from pathologies in the oro-buccal system, amongst such pathologies including gingivitis, peridontitis and dental calculus. The search for a therapeutic agent goes deep back in time. The principle of using a substance or a mixture of substances for cleaning teeth, preventing or treating teeth diseases is not a new concept. The terminology concerned to mouth care might be changed but there is clear evidence of dentifrices and mouthrinses on around the year 4000 B.C., that is, some 6000 years ago (1).

The routine and mechanic removal of upper gum dental plaque as a method of disordering the bacterial biofilm which is a part of gum plaque is considered as an efficient and decisive resource to prevent gum plaque from developing and to provide gum plaque treatment or preventing a comeback of gingivitis, periodontitis and dental calculus (mineralized plaque). There is in literature full evidence that proves the role of upper gum biofim as a primary reason for the development of such mouth diseases and as well as the importance of upper gum biofilm disorder and/or its removal for maintenance of dental and gum health. Likewise, it was proved in a plain way a relation that exists between the upper gum biofilm (gingivitis etiology) and the lower gum biofilm (periodontitis etiology) being indispensable the presence of the first one to cause the second to take place and to cause an evolution from gingivitis to periodontitis in an individual who is susceptible to this transition.

There is sufficient evidence in literature that proves the role of upper gum biofilm as being the primary cause for the development of said diseases (2) and as well as the importance of its disorder and/or removal to maintain the dental and gum health.

Furthermore, it was also clearly proved the relation that exists between the biofilm formed in the upper gum region (gingivitis etiology) and the biofilm formed in the lower gum region (periodontitis etiology) and the presence of upper gum biofilm being indispensable for the lower gum biofilm to take place therein and thus occuring an evolution from gingivitis to periodontitis in an individual who is susceptible of passing through this transition (4).

The biofilm formation is a very dynamic and well orderly process. In fact it is not, as it was believed to be, bacteria that was deposited on the teeth surface. In a surface of clean teeth the agents that form a primary biofilm, members of a Streptococcus species, will adhere first. The presence of such member is essential for the adhesion of other species of bacteria. Colonies that come next appear to supply the media in order to create a suitable ambient for the adhesion and spreading of other microorganisms. In the case that biofilm is not fought against it will grow its size and complexity by the adjacent region of gum thus inducing an inflammatory response. As the complexity and size of biofilm are directly related to each other under normal circumstances, it is presumed that both are necessary for the gingivitis to occur.

The upper gum biofilm control is an own exclusive burden of the person himself/herself, who ought to use toothbrush and instruments to effect the proper cleaning amongst his/her teeth. Removal of lower gum plaque with or without surgical interference is under the responsability of a dentist surgeon by using instruments to scrape out through the teeth once the patient by himself/herself has no ways to reach said lower gum plaque. However, a series of local and systematic conditions might determine a momentary or definitive damage to the ability of patient to carry out by himself/herself the mechanical control exerted at home of upper gum plaque. Amongst such circumstances we can mention the inability and/or lack of physical conditions, the surgical periodontal or conservative posttreatment periods after a bucco-maxillo-facial surgery from other causes (6).

Tooth decay, gingivitis and periodontitis are infectious diseases once they are caused by pathogenic microorganisms that are added to surface and develop into a bacterial plaque that becomes highly harmful. This will take place owing to the unbalance of a resident microbe colony inside the mouth cavity that results from an improper hygiene and from an immunodefiency and/or inability of hosting individual to provide its own defense.

In spite of being both gingivitis and periodontitis reversible diseases, and as well is tooth decay, not always harmony in mouth cavity is obtained when considering mouth health by a gum plaque mechanical control. In such a way antimicrobial substances can and, of course, ought to be employed in a preserving or therapeutically way in order to recover the microbe colony balance and thus provide that hosting individual ways of defense maintain the mouth health state.

The search to develop products for chemical control of upper gum bacterial plaque and as well lower gum bacterial plaque aims at finding an alternative to achieve the proper elimination of said plaques without, for such achievement, depending solely on mechanical methods of removing them.

Antiseptic and other topic antibacterial agents are products relatively recent used in an attempt to prevent and treat gingivitis, periodontal diseases (7) dental calculus and its periodical comeback since the beginning of the seventies and the application of several types of substances that were developed for the chemical control of the upper gum biofilm however did not achieve the necessary conditions for a chemical agent to prevent and to control the periodontal disease in a proved efficient way.

It is important to emphasize that antiseptic agents seem to provide an action that is considerably more preventive than therapeutic. Thus, agents that are more efficient do really inhibit the development of plaques and gingivitis, but they are limited or slow in action with regard to plaques and gingivitis that had already taken place.

The necessary conditions for a chemical agent to perform the control of the upper gum plaque with proved efficiency were claimed by Loesche in 1976 and continue to be the basic pillars in the critical analysis of the clinical employment of products aimed at achieving this objective. As for the author, the chemical agent ought to be:
1 Efficient against microorganisms that are responsible for gum inflammation
2 Able of being maintained inside the mouth in order to have sufficient contact time to act over the existing microbe colony and to hold inhibition of gum plaque formation for a prolonged period of time
3 Stable under ambient temperature during a considerable period of time; and
4 Safe when exposed to human beings

Presently the antiseptic agent that better fits the above conditions and is available in the market is the chlorexidine as a "gold standard" compared to the other substances developed to fight the formation of plaques and development of gingivitis (9).

Many means might be used for application of antiplaque agents but most of information is related to tooth paste and mouthrinses.

Dentifrice seems to be the most practical method and the one with a better relation concerning to costs and benefits for the chemical control of plaques for it is normally used by most of individuals and because it might contain a number of components in its formulation that influence and help the consistency, stability and the function of the product. Disabled might benefit on other methods of application (10).

### BRIEF DESCRIPTION OF THE INVENTION

The antiseptic agent and main biocide, used in these systems that are now disclosed, were selected from N-chloramines compounds and, more preferably, the T-chloramines, that will be analysed under a vision aimed at meeting the aforesaid requisites of Loesche's disclosures and as well when compared to the chlorexidine's "gold standard" and even more the innovating and inventive conditions that it bears by itself.

### ATRIBBUTES OF ANTISEPTIC AGENT AND BIOCIDE NOW INNOVATED IN THIS ORGANIC SYSTEM COMPARED TO LOESCHE'S CLAIMED DISCLOSURES

1. Efficiency against microorganisms: the T-chloramine is a salt, sodium N-chloro-para-toluenesulfamide, C₇H7ClNNaO₂S, a chemical combination formed by adding a small amount of ammonia to chlorine water and when it is placed in an aqueous solution it will ionize and thus forming T-chloramines ions.: CH₃-C₆H₄-SO₂-NCl (99.6%) hypochlorite ions OCIO, hypochlorite acid HClO or salts from this acid like NaClO, HClO and NaClO derivatives active chlorines that hold a biocide action by either an oxidating reaction or by protein hydrolysis reacting with organic material from live microorganisms of any type, penetrating through and/or breaking the cell walls of bacteria: gran positive, gran negative, fungi, viruses, microbacteria, yeasts in a vegetative form (spori) or not with which it starts contacts with thus destroying the cell materials or interrupting essential processes leading to the their inevitable destruction.

This oxidating and protein hydrolysis reaction kills the microorganisms either within an aerobic or an anaerobic ambient very fast even in low concentrations.

Due to the irreversibility of the oxidating reaction and hydrolysis there is no possibility for the microorganisms to create a resistance against the active principle. Its antiseptic equivalent is equal to 4.6 degrees, thus possible of being considered amongst strong antiseptic substances. Buckley considers it with antiseptic power. Buckley likewise considers it with an antiseptic power that is four times higher than that of hypochlorites and adds Buckley that in the presence of blood the substance is more active than the Dakin's liquid, having a longer lasting antiseptic powder. (14).

Gottardi calculated the theoretical composition of a T-chloramines aqueous solution and concluded that the following balance exists: (R= CH₃-C₆H₄-SO₂)
R-NClNa<= => R-NCl -+ Na + (T- chloramines ion + sodium)
R-NCl-+ H + <= =>R - NHCl (T-chloramines acid)
2 R-NHCl <= =>R- NHCl₂ (para-toluenesufonamide + T-dichloramines)
R-NCl₂ + H₂O <= => R-NHCl + HOCl (T-chloramines acid - T + hypochlorine acid)
HOCl <= => OCl- + H + (hypochlorite ion + hydrogen)
R-NCl- +H₂O <= => R-NH₂ + OCl (para-toluenesulfanamide+ hypoclorite ion)
R-NCl -+ H₂O <= => R-NH₂ + O + Cl - (para-toluenesulfonamide + free oxygen + chlorine ion)

In accordance with this balance seven different combinations might be present in a T-chloramines solution. It is estimated that these molecules represent over 99.99% (per cent) of total amount of T-chlorarmines in the solution.

The microorganisms that T-chloramines proved being active against are the following:
• Bacteria
• Viruses
• Fungi
• Seaweed
• Yeasts
• Parasite

### Bactéria

| | | |
|---|---|---|
| Acinetobacter spp | Escherichia coli | Salmonella durban |
| Actinobacillus Pleuropneumoniae | Flavobacterium Branchiophilum | Salmonella livingstone |
| Aeromanoas hydrophila | Flavobacterium Haematocrits | Salmonela newbrunswick |
| Aeromomas salmonicida | Klebsiella pneumosiae | Salmonella newport |
| Alcaligens faecalis | Listeria manicytogenes | Salmonella oranienburg |
| Bacillus antaecis | Micrococcus avium | Salmonella paratyphi B |
| Bacillus anthracis | Micrococcus citreus | Salmonella pullorum |
| Bacillus cereus | Micrococcus pyogenes | Salmonella rostock |
| Bacillus diphteria | Moraxella ssp | Salmonella senftenberg |
| Bacillus dysentery | Mycobacterium ayium | Salmonella thompson |
| Bacillus mesentericus | Mycobacterium lacticola | Salmonella typhiurium |
| Bacillus sbtilis spores | Mycobacterium minetti | Sarcina lutea |
| Bacillus subtilus | Mycrobacterium Pellegrino | Shigella boydii |
| Bacillus tuberculosis | Mycrobacterium phlei | Shigella sennei |
| Bacterium enteritidis | Mycrobacterium piscium | Staphylcoccus aureus |
| Bacterium paratyphi | Mycobacterium smegmatis | Staphylococcus aureus haemolyticus |
| Bacterium rhusopatheae | Mycobaterium vole Bacillus | Staphylococcus bag |
| Brucella abortus bang | Pasteurella | Staphylococcus paratyphosa B |
| Brucella suis | Pediococcus cerevisiae | Staphylococcus pyogeneses |
| Clostriduim welchii | Proteus mirabilis | Staphylococcus agalactiae |
| Clostridium bifermentas | Pseudomanas aeruginosa s | Streptococcus faecium |
| Clostridium tertium | Pseudommas fluorescens | Vibrio alginoliticus |
| Clostridium histolyticum | Pseudomanas fragi | Vibrio angullarum |
| Clostridium caloritolerans | Pseudomanas putida | Vibrio cholerae |
| Enterobacter aerogenes | Pseudomans putrefaciens | Vidrio harveyi |
| Enteroobacteriaceae citrobacter | Pseudomonas Pyocyana | Vibrio parahemolyticus |
| Enterobacteriaceae hagnia | Pseudomonas tolaasii | Vibrio salmonicida |
| Enterobacteriaceae klebsibela | Pyoacyaneus | Yersinia enterocolitica |
| Enterobacteriaceae serratia | Salmonella Dublin | Yersinia ruckeril |
| Enterococcus faecium | | |
| Adenovirus | Fowl pox virus | Poliovirus |
| African swine fever virus | Gumboro disease virus | Porcine parvovirus |
| Aujeszky virus | Hepatitis B virus | Pox virus |
| Avian reovirus | Hepatitus contagiosa Canine virus | Pseudo bird pest virus |
| Canine parvirus | Herpes virus | Rabies virus (fixed) |
| Celovirus | Human immuno-deficiency virus (HIV) | Retrovirus |
| Corona virus | Infectious bronchitis virus | Rhino pneumonic virus |
| Coxsackle virus | Infectious bursitis virus | South African pest virus |
| Diphteria virus | Infectious pancretic Necrosis | Swine fever virus |
| Ektromelie virus | Influenza virus | Systematic ectodermal and mesodermal |
| Enephalomycarditis virus | Irido virus (ASFV) | Açulo virus (SMBV) |
| Enteric cytopathogenic bovine orphan virus | Myxomatosis virus | Teschen virus |
| (ECBO) | New Castle Disease virus | Toga virus |
| European swin fever virus | Orthopox commune virus (vaccinia) | Vaccinia virus |
| Foot and mouth disease virus | Paramyxo virus | Vesicular swine disease virus |
| Fowl plague virus (NCD) | Picoma virus | White spot disease virus (SMBV) |

### Fungi

| | | |
|---|---|---|
| Aspergilus amstellodami | Microsporum gypseum | Algas |
| Aspergillus flavus | Myrothecium verrucaria | Anabaena cylindrica |
| Aspergillus fumigatus | Oospora lactis | Chlorella vulgaris |
| Aspergillus niger | Paecillomyces variotti | Oscillatoria tenuis |
| Aspergillus versicolor | Penicillium funiculosum | Skeletonema sp. |
| Chaetomium globosum | Penicillium verruccosum | Stigeoclonium sp. |
| Cladosporium cladosporoides | Saprolegnia parasítica | Tetraselmis sp. |
| Entomophthora destruens | Trichoderma viride | Yeasts |
| Entomophtora thatxteriana | Trichophytoon equinum | Candida albicans |
| Endomophothora virulent | Trichophyton mentagrophytes | Cryptococcus ssp |
| Epidemophyton floccosum | | Saccharomyces diastaticus |

Parasites
Epystylis
Girodactilus salaris
Ichtyobodo necator
Ichtyophthiriu

2. Substantiveness: by the fact that T-chloramines molecule holds an anion charge it is given to this agent a considerable substantiveness, thus providing a prolonged protection for it being able of adsorbing the bacterial biofilms, the organic matters and mouth structures, being slowly released in the mouth ambient once its action mechanism is activated in the presence of microorganisms, releasing an active group in a necessary proportion and thus maintaining bacterial static and biocide conditions on the saliva and in the dental plaque itself for a considerable period of time.

When applied as a therapeutics it acts as an antiseptic of prolonged action and is able of being useful for 24 hours (15).

The T-chloramines is a part of a group of oral antiseptics that exert a strong sustainable effect for at least one hour after its application and it is a mouthrinse indicated for being used in individuals/patients that were subject to chemical therapy and benefit from a continuous reduction in the bacterial flora in the oral cavity; however, said aseptic agents also might be used prior to the extraction of teeth (16). It is strongly absorbed by the electrostatic interaction between the negative charges of T-chloramines and the ions positive charges of apatita calcium and from carbon structures formed from organic compounds that are present in microorganisms and organic matters from bacterial plaque with a slow disadsorption in the surfaces, thus having a sufficient time for contact to act over the existing microbe colony with bacteria static and biocide action maintaining the inhibition of formation and/or development of plaques for a prolonged period.

3. Stability to ambient temperature: the antiseptic agent used in this system has an outstanding stability in an acqueous solution even under high temperatures. Tests that were carried out indicate that for a solution under 5%, stored in a opaque container under 25°C after a period of 210 days less than 0.05% of activity was lost due to decomposition. As for solutions under 0.5% after a period of 30 days under 40°C only 0.01% was then decomposed. When they were exposed in an ambient with organic matter and microorganisms after a period of 24 hours approximately half of aseptic agents were still active. T-chloramine ions are highly stable and will remain active for a long period of time (13).

4. Safe for exposure to human beings: T-chloramines have been used for treatment of potable water since the beginning of last century in the United States and, in some cases; for longer than uninterrupted 80 years, not being irritating to skin, to the eyes and to mucosas particularly the mouth mucosas used in the indicated dosages.

### Toxicant informations

Acute intoxication - LD50, oral, mouse -1000 mg/kg
Inhalation - LC 50 mouse > 0.275 mg/l (4 hours - maximum reached concentration)
Irritation - Skin, solution under 8% - not irritating
Eye - solution under 8% - moderately irritating
Solution under 0.5% - not irritating
Genotoxity Ames Test: Not mutagenic
Micronuleus Test: Not mutagenic

Subchronic Toxicologicant information: oral toxicity in mouse, after 90 days, level 15mg/kg/day- nothing was observed (14). It is found through these technical informations and through time period of use that the antiseptic agent is extremely safe to human beings within dosages of use provided in the systems.

### COMPARISON BETWEEN THE ANTISEPTIC AGENT AND BIOCIDE T-CHLORAMINES PRESENT IN THE SYSTEMS AND CHLOREXIDINE

The chlorexidine is a compound with strong and di-cations base with positive charges in each side of hexamethylene link that turns it highly interactive with anions, thus turning difficult its formulation in products. It inter-acts with anionic detergent thus not being able to be formulated with them. Furthermore, chlorexidine is incompatible with soaps and anionic compounds. It is difficult to be formulated for tooth paste for the aforesaid reasons (18).

The chlorexidine is neither a sporicide, neither a viruscide and nor active against microbacteria (19).

The chlorexidine has a certain number of side local effects. The most common and problematic one of them is the brownish colouring it imparts to teeth, to some resin materials of restoring works and dentures and to mucosas mainly located in the back of the tongue (21).

It is the pigment side effect that limits the use of chlorexidine for a long term in the preserving orthodontics and it occurs in the same way with products that were correctly formulated including gels and sprays (22). The chlorexidine has a bitter taste that hardly went on without being perceived and in many individuals it causes a disturb in the palate. The individual's taste for salted foods seems to be mostly affected in such a way to turn food somewhat tasteless (23). The chlorexidine increases the formation of upper gum calculuses and also, although rarely observed, increases the reversible swellings in the lips and/or in the salivary glands and as well as increases scalings off in the oral mucosa.

In spite of the excellent inhibiting properties of chlorexidine against plaques its prolonged and spreaded use is limited by its local side effects.

Furthermore, owing to the cation nature of chlorexidine and in consequence its low power of penetration, said antiseptic has a very limited value in the therapy of stablished oral conditions, including gingivitis (25).

The T-chloramines for its anionic nature and even having a very small amount of free chlorine has a formulation that is facilitated for pharmaceutical products (13). It is compatible with anionic detergents and it can be formulated with said anionic detergents, thus it is easy to provide formulations for tooth paste, mouthrinses and other solutions once they are compatible with other compounds of the systems.

The T-chloramines hold a biocide action against all forms of microorganisms including viruses and microbacteria in the vegetative form either spori or not.

The T-chloramines do not hold local side effects and its use in the dosage prescribed in the practice of medicine has confirmed to us the absence of any type of side effect concerning to pigment colouring and morphophysiological alterations in any oro-buccal structure even when it is used during prolonged periods of time thus becoming itself an excellent therapeutic, cleansing and preserving agent.

Because of its intense powder (even intenser than free chlorine) to penetrate into biofilm and thus inactivate the plaque's bacteria the T-chloramines disorder, release and prevent the pathogenic action of the upper and lower gum plaque (26).

Because of its anionic nature T-chloramines lowers the superficial tension and might be associated with other compounds that implement its action and the system's efficiency. Surfactants like lauryl sodium sulfate, fluor like sodium monofluorphosphate, the copolymers like methoxyethylene co-polymer and malic acid (GANTREZ Trade Mark) and the polyphosphates like tetrasoda pyrophospate which are active principles proved to be safe and widely employed in the pharmaceutical industry and not imparting side effects and morphophysiological alterations of any oro-buccal structure even when used during prolonged periods of time and additionally for not being available any reference with regard to cancer related action and/or risk to health in a vast consulted bibliography and specialized in clinical toxicology. The lauryl sodium sulfate with detergent, emulsifying, antiseptic and anionic surfactant properties acts by softening the surface deposits thus emulsifying and suspending the residues that are then removed from the teeth surface (27). Also the lauryl sodium sulfate decreases the superficial tension and penetrates and facilitates the penetration and activity of the other components in the system. The fluor widely used as an anticaries agent has both bacteriastatic and inhibiting action against bacterias' enzymes that are responsible for the glycolysis with the consequent decrease of polysacherides' formation that are responsible for the adhesion and formation of the plaque with a decrease in the acidgene of envolved acids within the decaying process.

The polyphosphate existing in the fluor's ions are of major utility once they form soluble complexes with the calcium, iron, magnesium and maganeze ions and aids to keep in suspension the particles that form the dental tartar in a way that said particles can be easily elimated by teeth brushing and washing thus preventing the tartar formation and tartar deposits on the teeth. The GANTREZ (Trade Mark) agent is a substantiveness agent that imparts to the system as a whole a major ability of acting and efficiency for a more prolonged period of time and in such a way that allows the dosage decrease of the active components in the system, thus making the system safer. These actions enhance the T-chloramine effect thus permiting that its dosage be extremely low and allows to the system a very good penetration opening access within irregularities, interpositions, surface and interior of several mouth tissues, interdental spaces, periodontal spaces and upper gum bags, thus allowing a profound interaction once microorganisms will develop not only within surfaces and irregularities, but as well will develop within the teeth lanes. As a consequence it is necessary that the anti-infectious agent spreads itself through all regions thus providing to the system now developed unmatched conditions for the preservation and the therapeutics of the several oro-buccal diseases.

### OTHER ACTIONS OF COMPOUND SYSTEMS BY THE ANTISEPTIC AGENT AND BIOCIDE AND THEIR ADJUVANTS

The systems hold effective actions in the interdental, lower and upper gum bacterial plaque and furthermore they are caries preventing in relation to decay and bactericide for the decay's agents. As they are slightly basic they balance the buccal mouth pH, control the acidgenic bacteria effects and neutralize the degradation of carbon hydrates thus preventing fermentation and converting fatty acids present in the organic matter into soap in such a way to form fatty acid salts (soap) and glycerol (trialcohol) thus deeply acting as a cleaning agent. They react with the organic matter contained in the mouth and provoke the release of the organic matter, they fight the odour bacteria and provide a deodorizing action in the mouth and eliminating the halitosis. Further, they dissolve the dead tissues. The restrospection of literature explains that the action of the organic tissue dissolution is due to the hypochlorous acid (HOCl) and to the hypochlorite (OCl) that act over the proteins thus carrying out the hydrolisis. The systems significantly retard the production of collagen and prolong the acute inflammatory response curing adversely by a secondary intent and as a consequence arising the recovering action of tissues and repair of lesions thus promoting their fast skinning and scar healing. The systems act in an efficient manner over the vasomotor phenomenons in the inflammation of oro-buccal mucosas providing a rapid decompression. They have a very good effect over the desensitizing of teeth hypersensibility once the systems coagulate superficially the Thomas' fibres and enhance the dentine permeability thus permitting by adsorption the sealing of tooth tiny channels by the calcium that is present in its composition. For all of this they provide effective results for the preservation and treatment of oro-buccal diseases, gingivitis, periodontitis and all of their forms and also providing effective results in the occurrances of chronical serious mouth diseases, periodontal bags, dental mobility even in the simplest form of hyperemia with wonderful and surprising therapeutic results once they react to eliminate the cause thus stimulating scar healing, accelerating the recovery process of harmed tissues, renewing the fibers of alveolus-dental union and providing the hardness of dental elements.

### OBJECTIVES OF THE INVENTION

Systems react fast; after their use the symptoms and occurrences of immunological and inflammatory reactions hold back in a period from 24 to 72 hours and this makes easier the start of clinical and surgical intervention. The biocide agent in the system, the T-chloramine, was defended in discussion as a surgical periodontal adjuvant, and when surgeries are preceded by use of the systems in the therapeutics and/or anti-sepsis results prove that the tissue recovery will take place in a faster manner thus decreasing significantly the occurrence of bacteremia post-surgery. Clinical advantages for the patient can also be proved in view of a less painful post-surgical situation such a patient status that comes to be better tolerated. The post-surgical edema is insignificant or absent when compared to cases in which the systems are not previously used. Besides such advantages the surgical status shows itself anti-sepsised and as well as in better conditions during the surgical act once the use of the systems highly decrease the bleeding and surgical time.

The systems efficiently act as oral cleansing elements and local preservation and therapeutics in the case of more diversified oro-buccal pathologies such as:
a) Painful and swelling inflammatory processes
b) Dental hyper sensibility
c) Odonto-stomachic diseases: gingivitis, periodontitis, ulcerations, tongue inflammation, stomach diseases, mucosa ulceration
d) Otolaryngologic diseases: laryngitis, pharyngesitis, angina and so on
e) Oral hygiene and benefits to gum health for the physical and mental disable
f) people and for patients under mandibular treatment
g) Individuals who are by their immunology or by use of medicine effected themselves and prone to oral infections
h) Patients that undergo radio and chemical therapy treatments
i) Patients that have a high risk of caries and/or patiients that are exerting preservation and therapeutics on caries
j) Individuals that are users of dentures and dental fixed or removable appliances
k) Irrigation and/or mouth rinse in the pre-surgical or post-surgical asepsis for surgical proceedings like implants, mandible's fracture, alveolus disease prevention, accidental traumatic ulcerations and so on
l) Reduction of post-surgical and post-exodontia bacteremias
m) Halitosis, gum bleeding and tongue plaques control.
n) Reduction of pathogenecity for aerosols
o) They (systems) can and even should be used for replacement of normal dentifrices or rinsing agents used in daily mouth asepsis and for controlling of chronic and acute pathologies once the systems act as preventing and healing agents and avoid comeback of oral buccal diseases without, however, intervening in the microbe colony balance but, instead of it, mitigating the harmful effects of bacteria that are significantly pathogenical by the quantity control of said bacteria and disorder of teeth plaque.

In such a technical context as described above, one of the essential objectives of the present invention is to provide a never offered before system that, formulated as a dentifrice, cream or gel, mouthrinse, therapeutic gel, solution or cream, that has the following properties: biocide, anti-caries, anti-inflammatory, bacterial and dental anti-plaque, halitosis deodorant, dental desensetizing agent and cleansing agent, used particularly in hygiene care, preservation care and therapeutic care in the several oro-buccal diseases.

Another objective of the present invention is to disclose an antiseptic, biocide and cleansing active principle presented as a system that can be formulated with other compounds and mainly with other compounds with enhancing and adjuvant properties of the effects and actions of this active principle.

Another fundamental objective of the present invention is to provide to the health field practitioners a new product with no existing similar in the market.

Another essential objective of the present invention is to provide a system whose preparation process be an industrial process, less costly and a system that can be easily processed.

In order to achieve their objectives the inventors searched and prepared experiments in a very incisive way seeking an antiseptic and biocide agent which would meet all the aforesaid requisites ending at last in what is concerned with the required proofs and in a thoroughly surprising way by the revelation of N-Chloramines, and specially the T-Chloramine.

As far as it is concerned with proofs of properties, actions and effects in the systems, the inventors by themselves and by professionals acting in the odontology area had tested and carried out innumerous experiments proving the attributes of the systems now disclosed.

### DESCRIPTION OF THE INVENTION AND FORMULATIONS

The pharmaceutical composition in the invention might be prepared by the mixture of the suggested components, or compatible similar components as, for instance, as disclosed in the formulas I, II, III, IV and V of this invention that are disclosed next, by employing the conventional techniques known by the technicians in the area but taking into consideration the percentage dosages in weight relating to total mass, mainly in regard with the chief active principle, a derivative of N-Chloramines T-Chloramine which ought to have its percentage in weight with relation to the total mass never under 00.1% and never over 4.00%.

The disclosure of the invention is concluded with the systems that were described previously in a patent application and/or similar use in therapeutics and in the medical, pharmaceutical and odontological markets with the presentation of examples cited in formulations I, II, III, IV and V.

### FORMULA I: PASTE OR GEL DENTIFRICE

List of products significantly used:
T-Chloramine, salt, sodium N-chloro-para-toluenesulphanamide used with a percentage of weight in relation to the total mass never lower than 0.01 % and never higher than 4.00%.
Sodium sulfate lauryl, CH₃(CH₂)₁₀CH₂OS₃NaS, used with a percentage of weight in relation to the total mass weight between from 0.10% to 2.00%, or a compatible similar product.
Tetrasoda pyrophosphate used with a percentage of weight in relation to the total mass between from 0.10% to 2.00% or a compatible similar product.
Methoxyethylene co-polymer and malic acid (GANTREZ, Trade Mark) used with a percentage of weight in relation to the total mass between from 0.10% to 2.00% or a compatible similar product.
Sodium monofluorphosphate used with a percentage of weight in relation to the total mass between from 0.10% to 2.00% or a compatible similar product.
Calcium carbon, CaCO₃, used with a percentage between from 4% to 16% in relation to the total weight of mass or a compatible substitute.
Glycerin used in an approximate percentage of between 1% to 10% in relation to the total weight of mass or a compatible product.
Glycerin used in an approximate percentage of between 0.50% to 30.00% in relation to the total weight of mass or a compatible product.
Hydroxypropylmetylcelullose, used in an approximate percentage of between 0.10% to 4.00% in relation to the weight of total mass or a compatible substitute.
Titanium dioxide used in a percentage of weight in relation to the total mass between from 0.10% to 2,50% or a compatible similar product.
Sodium benzoate used in a percentage of weight in relation to the total mass between from 0.10% to 1,00% or a compatible similar product.
Glycerin used in a percentage of weight in relation to the total mass between from 0.10% to 8.00% or a compatible similar product.
T chloramines, salt, sodium N-chlorine-para-toluenesulphanamide with a percentage in relation to the weight of total mass lower than 0.1% and the highest at 4%.
Sodium sulphate lauryl, CH₃(CH₂)₁₀CH₂OS₃NaS, used in an approximate percentage between from 0.1% to 2% in relation to the weight of total mass or a compatible similar.
Saccharine, used in a percentage between 0.01% to 0.5% in relation to the weight of total mass or a compatible similar product.
Saccharine, used in a percentage of weight in relation to the total mass between 0.01% to 1.00% or a compatible similar product.
Mint essence used in a weight percentage in relation to the total mass between 0.10% to 2.00% or a compatible similar product.
Menthol, used in an approximate percentage between 0.1% t0 1.6% in relation to the weight of total mass or a compatible similar product.
Menthol used in a weight percentage in relation to the total mass between 0.10% to 3.60% or a compatible similar product.
Deionized water, qsp, 100% in weight

### FORMULA II: MOUTHRINSE

List of products significantly used:
Calcium carbonate, CaCO₃, used in an approximate percentage between 0.1% to 5% in relation to the weight of total mass or a compatible similar.
Sorbitol, with a percentage between 0.3% to 2.8% in relation to the total weight of mass or a compatible similar.
T chloramines, salt, sodium N-chloro-para-toluenesulphanamide, C₇H₇ClNNaO₂S, with a percentage in relation to the total weight of mass never lower than 0.01% and not higher than 4.00%
Lauryl sodium sulphate, CH₃(CH₂)₁₀CH₂OS₃NaS, used with a percentage between 0.2% to 5% in relation to the total weight of mass or a compatible similar.
Lauryl sodium sulphate, CH₃(CH₂)₁₀CH₂OS₃NaS, used with a percentage in relation to the weight of total mass between 0.10% to 2.00% or a compatible similar
Tetrasoda pyrophosphate used with a percentage in weight in relation to the total mass between 0.10% to 2.00% or a compatible similar product.
Methoxyethylene co-polymer and malic acid (GANTREZ, Trade Mark) used with a percentage in weight in relation to the total mass between 0.01% to 2.00% or a compatible similar product.
Sodium monofluorphosphate used with a percentage in weight in relation to the total mass between 0.10% to 2.00% or a compatible similar product.
Hydroxyethylcelullose, used with a percentage in weight in relation to the total mass between 0.10% to 2.00% or a compatible similar product.
Glycerin, used with a percentage in relation to the weight of total mass between 1.00% to 20.00% or a compatible similar.
Saccharine, used with a percentage between 0.01% to 0.30% in relation to the total weight of mass or a compatible similar product.
Nipagin used with a percentage in relation to the weight of total mass between 0.01% to 0.50% or a compatible similar product.
Nipazol used with a percentage in relation to the weight of total mass between 0.01% to 0.50% or a compatible similar product.
Menthol, used with a percentage in weight in relation to the total mass between 0.10% to 3.6% or a compatible similar product.
Demineralized water, qsp -100%

### FORMULA III: ANTISEPTIC SOLUTION AND BIOCIDE - THERAPEUTIC GEL

List of products significantly used:
Hydroxyethylcellulose, used with a percentage in relation to the weight of total mass between 0.01% to 3.00% or a compatible similar product.
T-chloramines, salt, sodium N-Chloro-para-toluenesulphanamide, C₇H₇ClNNaO₂S, used with a percentage in relation to the total weight of mass never lower than 0.01% and never higher than 4.00%.
Sodium sulphate lauryl, CH₃(CH₂)₁₀CH₂O₂S₃NaS, used with a percentage between 0.1% to 3% in relation to the total weight of mass or a compatible similar.
Sodium sulphate lauryl, CH₃(CH₂)10CH₂O₂S₃NaS, used with a percentage in weight in relation to weight of total mass between 0.10% to 2.00% or a compatible similar product.
Methoxyethylene co-polymer and malic acid (GANTREZ, Trade Mark), used with a percentage in weight in relation to the total mass between 0.01% to 2.00% or a compatible similar product.
Hydroxyethylcelullose, used with a percentage in relation to the weight of total mass between 0.01% to 3.00% or a compatible similar product,
Glycerin, used with a percentage in weight in relation to the total mass between 0.10% to 30.00% or a compatible similar product.
Nipasol, used with a percentage in relation to the weight of total mass between 0.01% to 0.50% or a compatible similar product.
Menthol, used with a percentage in weight in relation to the total mass between 0.10% to 3.6% or a compatible similar product.

### Demineralized water, qsp -100%

### FORMULA IV: ANTISEPTIC SOLUTION AND BIOCIDE

List of products significantly used:
T-Chloramines, salt, sodium N-Chlorine-para-toluenesulphanate, C₇H7CINNaO₂S, used with a percentage in weight never lower than 0.01% and never higher than 4.00%
Sodium sulphate lauryl, CH₃(CH₂)₁₀CH₂OS₃NaS, used with a percentage between 0.01% to 2.00% in relation to weight of total mass or a compatible similar.

### FORMULA V: CLEANSING CREAM FOR REMOVABLE DENTURES

List of products significantly used:
T-Chloramines, salt, sodium N-chloro-para-toluenesulphanate, used with a percentage in weight in relation to the total mass never lower than 0.01% and never higher than 4.00%
Sodium lauryl sulphate, CH₃(CH₂)₁₀CH₂OS₃NaS, used with a percentage in weight in relation to the weight of total mass between 0.10% to 2.00% or a compatible similar product.
Tetrasoda pyrophosphate, used with a percentage in weight in relation to the total mass between 0.10% to 2.00% or a compatible similar product.
Methoxyethylene co-polymer and malic acid (GANTREZ, Trade Mark) used with a percentage in weight in relation to the total mass between 0.10% to 2-00% or a compatible similar.
Calcium carbonate, used in a percentage between 2.00% to 35.00% in relation to the weight of total mass or a compatible substitute.
Glycerin, used with an approximate percentage between 0.50% to 30.00% in relation to weight of total mass or a compatible product.
Hydroxypropylmetylcelullose, used with an approximate percentage between 0.10% to 4.00% in relation to the weight of total mass or a compatible substitute.
Titanium dioxide used with a percentage in weight in relation to the total mass between 0.10% to 2.50% or a compatible similar product.
Sodium benzoate, used with a percentage in weight in relation to the total mass between 0.10% to 1.00% or a compatible similar product.
Glycerin, used with a percentage in weight in relation to the total mass between 0.10% to 8.00% or a compatible similar product
Saccharine, used with a percentage in weight in relation to the total mass between 0.01 % to 1.00% or a compatible similar.
Mint essence, used with a percentage in weight in relation to the total mass between 0.10% to 2.00% or a compatible similar product.
Menthol, used with a percentage in weight in relation to the total mass between 0.10% to 3.60% or a compatible similar product.

### Demineralized water, qsp -100% in weight

The organic systems of the present invention are preferably featured by the fact that said compositions in the systems (A), (B), (C), (D), (E) can hold, besides its main active principle, said antiseptic and biocide agent in an aqueous solution selected amongst the N-chloramines, other adjuvant agents selected from:
- The natural and/or artificial sweetening agents like saccharine and its calcium, potassium and sodium salts, derivatives of ciclamic acid and its calcium, potassium and sodium salts, sucralose, manitol, xilitol, sorbitol and/or steviosideos derivatives.
- The flavouring agents amongst natural, artificial, aromatic vegetables and/or those one chemically defined, pure or mixed.
- The natural and/or artificial colouring agents like those food colouring blue (3), yellow (4), green (3) DC red (33), titanium dioxide and others
- Refreshing agents like mint, menthol and/or eucalyptol
- Moistening agents like glycerin.
- The thickening agents that belong to the celluloses, gums, carbopol gels, silica gels.
- The preserving agents like the metylparabeno, propylparabeno, derivatives of benzoic acid, calcium benzoate, sodium benzoate, and/or potassium benzoate. The abrasive agents like silicium (Si) silicium dioxide, bi-calcium phosphate and those ones from carbonate families like calcium carbonate and/or sodium bicarbonate.
- The anti-caries derived from fluor like the sodium monofluorphosphate and/or sodium fluoride.
- The detergents like sodium sulfate lauryl, sodium sulfate and/or sodium sarcozinate lauryl.
   The substantiveness agents like the derivatives from malic acid (Gantrez).
- The anti-tartar agents that belong to the family of monosoda disoda or trisoda like sodium pyrophosphate and/or potassium pyrophosphate.
- The antibacterial agents like zinc chloride, cetylpyridineum chlorideand/or triclosan.

When the composition is formulated as (A) a dental paste, the composition is preferably a mixture of said antiseptic and biocide agent and said surfactant agent, anti-caries, anti-tartar, substantiveness agent and, additionally an abrasive agent, a moistening agent; a thickening agent; and an anti-tartar agent; and as well as one or more functional additives selected from a group that comprises flavouring agents, colouring agents and refreshing agents.

When the composition is formulated as (B) a mouth rinse, the composition is preferably a mixture of said antiseptic and biocide agent and said surfactant agent, anti-caries, anti-tartar, substantiveness agent and, additionally, a moistening agent, a preserving agent and a thickening agent; and as well as one or more functional additives selected from a group that comprises flavouring agents, sweetening agents and refreshing agents.

When the composition is formulated as (C) a solution therapeutic gel, the composition is preferably a mixture of said antiseptic and biocide agent, said surfactant agent, said substantiveness agent, and, additionally, a moistening agent, a preserving agent and a thickening agent, and as well as one or more functional additives selected from a group that comprises flavouring agents, sweetening agents and refreshing agents.

When the composition is formulated as (D) a preserving solution, the composition is preferably a mixture of said antiseptic and biocide agent and said surfactant agent, and, additionally, a moistening agent, a preserving agent and a thickening agent, and as well as one or more functional additives selected from a group that comprises flavouring agents, sweetening agents and refreshing agents.

When the composition is formulated as (E) a cleansing cream for removable dentures, the composition comprises a mixture of said antiseptic and biocide agent, said anti-tartar agent, said substantiveness agent, and, additionally, an abrasive agent, a moistening agent, and a thickening agent; and as well as one or more functional additives selected from a group that comprises a flavouring agent, a sweetening agent and a refreshing agent.

In spite of the invention being disclosed in full details, it is important to understand that the same does not limit its application to the drawings and steps mentioned herein. The invention is able of being available in other modes and as well of being carried out or practised in a plurality of ways. It should be understood that the terminology that was employed herein is for the purpose of description and not for limitation.

### CONSULTED BIBLIOGRAPHY

(1) Fischman, S.L. (1992) Hares theeth to fluorides; historical of dentifrice use. In: Embery, G & Rolla, G., eds. Clinical and biological aspects of Dentifrice, Oxford: Oxford University Press, pp.1-7
(2) Loe et al. Experimental gingivitis in man. J Pperiodonti, v.36, p.117/87.1965
(3) Wunderlich, R.C. et al. The therapeutic effect of toothbrushing on naturally occurring gingivitis, J Am Dent Assoc, v. 110, 1.6, p.929-31, Jun 1985
(4) Van Dyke T.E. et al. What is gingivitis ? Current understanding of prevention, treatment, measurement, pathogenesis and relation to peridontitis J Int acad Periodontal, v.1, p.000-000, 1998
(5) Keiser, J.B. (1994) Non-surgical periodontal therapy, In: Lang, N.P. & Karring.T. eds. Proceedings of the 1st European Workshop on Periodontology London Quintessence, pp 131-158
(6) Newman, Mg.: Korman, K.S. O uso de antibiótico e antimicrobianos na prática odontológica. São Paulo, Quintessence, 1997.
(7) Germom, P. et al. The plaque-inhibiting capacity of 11 antibacterial compounds. J. Peirodont Res, v. 5. N.2, p. 102-109, 1970
(8) Pillon, F.P., Avaliação crítica dos recursos químicos para o controle da placa bacteriana supragengival p.105-116, 2001
(9) Jones, C-G Chlorhexidine: is it still the gold standard ? Periodontal 2000, v.15, p.55-62. oct 1997
(10) Addy, Martin, lindhe, Jan, tratado de periodontologia clinica e implamentalogia oral, cap. 16, p.333-345-1996 TRADUZIR
(11) W.Gottardi, arco.Pharm., 325, 377-384, 1992
(12) M.Hahn, H.H. Ruttinger, R. Thamm, anal.Chin Acta, 289.35-42, 1994
(13) Akzo nobel boletim tecnial: Halamid the universal disinfectant, 1995
(14) Salles, E. Cunha, Terapêutica, Ed. Scientífica, p.93-95,1945
(15) Belissário, P.B., Farmacologia introducción a la terapéutica adontologica p. 66-58,1951
(16) F.A. Pitten - A. Kramer, Eur. J. Clin Pharmaco (1999) 55: 95-100. antimicrobial efficacy of antiseptic mouthrinse solutions.
(17) Barkvoll, P., Rolla, g & Svebdsen, A. 1989 Intraction between chlorexidine digluconate and sodium lauryl sulphate in vivo. Journal of clinical Periodontology 16, 593-598
(18) Sanz, M. Valicorba, N., Fabregues, S, Muller, I & Herkstroter, F. 1994. the effects of a dentifrice containing chlorexidine and zinc on plaque, gingivitis, calculus and tooth stining. J. of clinical Periodontology 21, 431-437.
(19) Silva, Penildon. Farmacologia, Ed. Guanabara, p. 1146-1150, 1998
(20) Flotra, I. Germo et al, side effects of chlorexidine mouthwashes, Scandinavian Journal of Dental Research, 79p. 199-125, 1971
(21) Eriksen, H, M., Kantanen, H. & Elligsen, J.E. Chemical plaque control and extrinsic tooth discolouration. A review of possible mechanisms. Journal of clinical periodontology. 12, 345+350 -1985
(22) Renton-Harper, P, R., Milson, S., Wade, W.G.Addy, M.Moran, J. & Newcomb, R.G., an approach to efficacy screening of mouthrinses: study on a group of French products (11) Inhibition of salivary bacteria and plaque in vivo, Journal of clinical periodontology 22, 723-727, 1995
(23) Lang N.P., Catalonotto, F.A. Knopfli, R.U. & Antezak, A A A A, Quality specific taste impalerment following the application of chloroxedine glucanate mouthrinses, Journal of Clinic periodontology 15, 43-48, 1988
(24) Flotra, L et al. Side effects of chlorexidine mouth washes, Scand J. Dent Res, v.p. 119-125,1971
(25) Addy, M. chlorexidine compared with other locally delivered antimicrobials. A short review. J. of clinical periodonology 13, 957-964 - 1986
(26) Le hevaller M.W., cawthon C.D., Lee R.G., Environ Microbial 1988 Oct., 54 (10): 2492
(27) Newbrum, Ernest. Tratado de Cariologia. 2 Ed. P.287-288, 1988
(28) Moorer, W.R; Wesselink, P.R. Factors promoting the tissue dissolving caoability of sodium hipoclorite. Int. Endodontic J. v. 15. N. 4, p. 187-196, oct 1982
(29) Brenan, S.S.M.E. Nutr, Leaper DJ. Diário de Infecção hospitalar JC:id68(3):263-267,1986 Nov
(30) Rams, TE., W.E., Keyes, P.H. and Houward, S.A.Lang., Effects of microbiologically modulatec periodontal therapy on advanced adult periodontites. Journal of the American Dental Association. 111: p. 429-441, 1985
(31) Sweet, J.B., Gil, V.J., Nitroblue Tetrazoliun, and Limulus assays for bacteremia at the dental extraction: effects of topical antiseptics. Journal of the American Dental Association. 96: P. 276

## Claims

1. A composition that is formulated as (A) a dental paste, (B) a mouthrinse, (C) a solution therapeutic gel, (D) a preserving solution or (E) a cleansing cream for removable dentures, comprising:
(1) an antiseptic agent and biocide in a aqueous solution selected from the N-chloramines, preferably the T-chloramines, with a concentration (CII) maximum of 4% by weight in relation to the total mass of the composition; and
(2) an anionic surfactant agent selected preferably within the group of tensoactive formulations such as sodium sulphate ether diethyleneglycol lauryl and sodium sulphate lauryl with a concentration (CIII) maximum of 3% by weight in relation to the total mass of the composition.

2. A composition as claimed in claim 1, wherein the antiseptic agent and biocide has a concentration CII of less than 2% by weight, preferably in the range of from approximately 0.01 % to approximately 1% by weight in relation to the total mass of the composition.

3. A composition as claimed in claim 1 or claim 2, wherein the anionic surfactant agent has a concentration CIII in the range of from approximately 0.011% to approximately 3% by weight, optionally approximately 2% by weight in relation to the total mass of the composition.

4. A composition as claimed in any preceding claim, further comprising an active agent with anti-caries action, preferably sodium monophosphate, having a concentration (CIV) maximum of 2% by weight in relation to the total mass of the composition; wherein, optionally, the active agent has a concentration CIV in the range of from approximately 0.01% to 2%, further optionally, approximately 1.25% by weight in relation to the total mass of the composition.

5. A composition as claimed in any preceding claim, further comprising
an anti-tartar additive agent selected preferably from the group of soluble pyrophosphates, more preferably sodium tetrapyrophosphate with a concentration (CV) maximum of 2.% by weight in relation to the total mass of the composition; wherein, optionally, the anti-tartar agent has a concentration CV of from approximately 0.01% to 2%, further optionally approximately 1% by weight in relation to the total mass of the composition.

6. A composition as claimed in any preceding claim, further comprising a substantiveness additive agent, preferably being a co-polymerand, more preferably methoxyethylene and polyvinylmethyl maleic acid with a concentration (CVI) maximum of 2.% by weight in relation to the total mass of the composition; wherein, optionally, the agent has a concentration CVI of from approximately 0.01% to 2%, and further optionally approximately 0.50% by weight in relation to the total mass of the composition

7. A composition as claimed in any preceding claim when formulated as a dental paste, further comprising:
1a) an abrasive agent
1b) a moistening agent
1c) a thickening agent; and
1d) an anti-tartar agent

8. A composition as claimed in claim 7, further comprising:
one or more functional additives selected from a group that comprises:
- flavouring agents
- colouring agents
- refreshing agents.

9. A composition as claimed in any one of claims 1 to 6, when formulated as a mouth rinse, further comprising the following agents:
- a moistening agent
- a preserving agent
- a thickening agent.

10. A composition as claimed in claim 9, further comprising one or more functional additives selected from a group that comprises the following:
- flavouring agents
- sweetening agents
- refreshing agents.

11. A composition as claimed in claim 6, when formulated as a solution therapeutic gel, further comprising
- a moistening agent
- a preserving agent
- a thickening agent.

12. A composition as claimed in claim 11, further comprising one or more functional additives selected from a group that comprises the following:
- flavouring agents
- sweetening agents
- refreshing agents.

13. A composition as claimed in any one of claims 1 to 6, when formulated as a preserving solution, further comprising:
- a moistening agent
- a preserving agent
- a thickening agent.

14. A composition as claimed in claim 13, further comprising one or more functional additives selected from a group that comprises the following:
- flavouring agents
- sweetening agents
- refreshing agents.

15. A composition as claimed in any one of claims 1 to 6, when formulated as a cleansing cream for removable dentures, further comprising
- an abrasive agent
- a moistening agent
- a thickening agent

16. A composition as claimed in claim 15, further comprising one or more functional additives selected from a group that comprises:
- a flavouring agent
- a sweetening agent
- a refreshing agent.

17. An organic system comprising a composition as claimed in any previous claim.

18. An organic system as claimed in claim 17, further comprising H₂O deionized qsp 100%.

19. An organic system as claimed in claim 17 or 18, further comprising one or more functional adjuvant agents.
